# EUROPEAN PATENT APPLICATION

(11) **EP 1 897 537 A1**
(43) Date of publication of application: **12.03.2008**
(21) Application number: 06120038.2
(22) Date of filing: 04.09.2006
(51) Int. Cl.: A61K 9/16, A61K 9/00, A61K 9/20, A61K 9/28, A61K 9/50

(54) **Composition comprising an angiotensin II receptor antagonist**

(71) Applicant: Novartis AG, 4056 Basel (CH)
(72) Inventor: The designation of the inventor has not yet been filed
(74) Representative: Hillebrand, Dirk

(57) **Abstract**

The present invention concerns pharmaceutical compositions comprising an acidic drug compound which is an angiotensin II receptor antagonist or a releasable form thereof, and a pH modifier.

## Description

The present invention relates to solid pharmaceutical compositions comprising a drug compound with a pH-dependent absorption profile. In particular, the invention relates to a pharmaceutical composition comprising an acidic drug compound, for example valsartan, which may be in the form of the free acid, a prodrug, a pharmaceutically acceptable salt, solvate, a polymorph, or a combination thereof. The acidic drug compound may be in a crystalline, partially crystalline, amorphous or liquid state.

Valsartan, i.e. (S)-N-(1-carboxy-2-methylprop-1-yl)-N-pentanoyl-N-[2'-(1H-tetrazol-5-yl)biphenyl-4-ylmethyl]amine, is a weakly acidic drug compound. The structure, preparation and formulation of valsartan is described for instance in US 5399578, US 6294197, WO 97/49394, WO 00/38676 and WO 01/97805, the contents of which are hereby incorporated into the present application by reference. Valsartan is an angiotensin II receptor antagonist and is effective and well tolerated in the treatment of congestive heart failure and reduction of blood pressure. Its combination with hydrochlorothiazide (HCTZ) is also known for the treatment of hypertension.

Valsartan is currently marketed as an immediate release tablet formulation (Diovan ®) containing valsartan 40 mg, 80 mg, 160 mg or 320 mg. Valsartan shows a low bioavailability (around 30 %) and relatively high inter- and intra-subject variability when administered in this form. Peak plasma concentration is reached around 2 to 4 hours after dosing, while the maximum hypertensive effect is usually achieved within 6 hours. A consequence of this is that the antihypertensive effect of a valsartan tablet taken in the evening may be substantially diminished by the morning; this is not ideal since blood pressure and heart rate are known to peak in the early morning hours. There is, therefore, considerable interest in providing a valsartan formulation which provides sustained release of the drug over longer periods of time, e.g. up to 24 hours from administration. There also exists a need for a valsartan formulation which results in one or more of reduced inter-subject variability, reduced intra-subject variability and increased bioavailability.

Acidic drug compounds such as valsartan exhibit significant pH-dependent permeability along the gastrointestinal (Gl) tract. The net charge of acidic drug compounds increases with pH, which in turn leads to a decrease in lipophilicity. A consequence of this is that the permeability of acidic drug compounds across the lipophilic membranes of the GI tract decreases with pH. Since pH gradually increases along the Gl tract (approximate ranges of pH values are: stomach pH 1 to 2; duodenum pH 4 to 5; jejunum and ileum pH 5 to 8; and colon pH 6 to 8), the permeability of acidic drug compounds decreases as the compounds travel along the intestine. This drop in permeability limits the rate of absorption and bioavailability of the compounds in the less acidic regions of the GI tract. Another property of acidic drug compounds is that their solubility in water increases with pH. As a result, the compounds are more soluble in the less acidic regions of the GI tract, increasing the likelihood that they will be excreted rather than absorbed.

The present invention is predicated at least in part on an appreciation that by shifting the microenvironmental pH of an acidic drug formulation to more acidic conditions, the net charge of drug molecules passing through the less acidic regions of the GI tract can be reduced. This enhances the lipophilicity and, in turn, the permeability of the drug molecules in these less acidic regions. Modulating the pH in this way also reduces the water solubility of the compounds in these regions, reducing the likelihood of the compounds being excreted. The invention is particularly relevant to formulations of weakly acidic drug compounds, especially valsartan and other angiotensin II receptor antagonists.

Accordingly, in one aspect the present invention provides a pharmaceutical composition comprising:
(i) at least one acidic drug compound which is an angiotensin II receptor antagonist or a releasable form thereof;
(ii) at least one pH modifier; and
(iii) at least one pharmaceutically acceptable excipient.

In a further aspect the present invention provides the use of an acidic angiotensin II receptor antagonist or a releasable form thereof and a pH modifier for the preparation of a medicament for the treatment of patients with disorders associated with angiotensin II receptor activity, especially by receptor inhibition.

In a further aspect the present invention provides a method of orally administering an acidic drug compound which is an angiotensin II receptor antagonist or a releasable form thereof, said method comprising orally administering to a patient in need of therapy a pharmaceutical composition according to the present invention. The composition may be in a form suitable for once a day administration.

Another aspect is an oral valsartan composition adapted to create a locally acidic environment in the intestine. The valsartan may be in the form of the free compound or a valsartan-releasing derivative thereof.

A further aspect concerns the use of a pharmaceutically acceptable acid for the manufacture of an oral medicament comprising an acidic drug compound which is an angiotensin II receptor antagonist or a releasable form thereof, wherein the pharmaceutically acceptable acid promotes protonation of the acidic drug compound in the intestine.

Further included is a method of enhancing the intestinal absorption of an acidic drug compound which is an angiotensin II receptor antagonist, comprising co-administering the acidic drug compound or a releasable form thereof with a pH modifier.

These and other features, advantages and objects of the present invention will be further understood and appreciated by those skilled in the art by references to the following specification and claims.

### Brief Description of the Drawings

Fig. 1 shows the dissolution profiles of (A) valsartan and (B) fumaric acid when present in the composition of Example 10.
Fig. 2 shows the dissolution profiles of (A) valsartan and (B) fumaric acid when present in the composition of Example 11.
Fig. 3 shows the dissolution profiles of (A) valsartan and (B) fumaric acid when present in the composition of Example 13.
Fig. 4 shows the dissolution profiles of (A) valsartan and (B) fumaric acid when present in the composition of Example 18.

### Detailed Description of Invention

### Acidic drug compound

The invention involves the use of acidic drug compounds which are angiotensin II receptor antagonists, or releasable forms thereof. As used herein the term "acidic drug compound" includes reference to drug compounds comprising one or more (e.g. one or two) acidic groups, for example one or more groups selected from carboxylic acid and tetrazole groups. The acidic drug compound may be a weakly acidic drug compound. In particular, this term includes reference to weak acids, for example comprising one or more acidic groups having a pKa of over 2, e.g. 2.1, for example 3 or more, 3.5 or more, and especially 4 or more. Of particular mention are angiotensin II receptor antagonists comprising one or more acidic groups carboxylic acid groups, for example valsartan, losartan, eprosartan, candesartan, telmisartan, irbesartan, olmesartan and the following compounds:

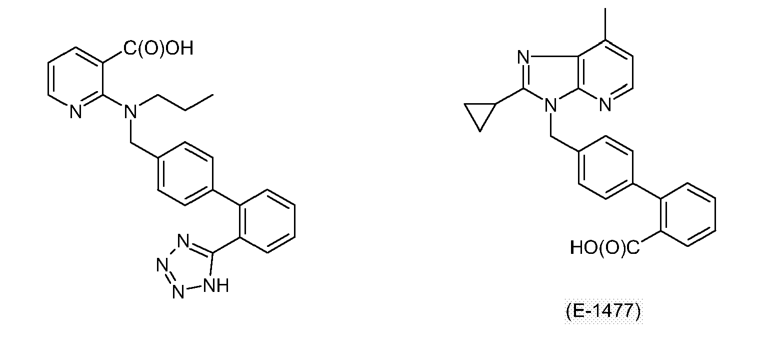

The drug compound may in a releasable form, the term "releasable form" as used herein referring to a form which is capable of releasing the acidic compound in, or delivering it to, the intestine upon oral administration in a controlled manner. The acidic drug compound may be in the form of the free acid, a prodrug, a pharmaceutically acceptable salt, solvate, a polymorph, or a combination thereof. By way of example, an acidic drug compound may be administered as a base addition salt which, in the conditions of the intestine, will result in formation of the free acid drug compound in equilibrium with at least one deprotonated form thereof, resulting in a specific time-dependent release profile. The drug compound may be in a crystalline, partially crystalline, amorphous or liquid state.

A preferred drug compound is valsartan or a releasable form (e.g. a pharmaceutically acceptable salt or dosage form) thereof. Of particular mention is valsartan free acid or a calcium salt thereof. The carboxylic acid group of valsartan has a pKa of about 3.9, while the tetrazole group of valsartan has a pKa of about 4.7.

The acidic drug compound may be used in combination with one or more other therapeutic agents. For example, a valsartan composition of the invention may further comprise one or more antihypertensive agents, e.g. hydrochlorothiazide (HCTZ).

Compositions of the invention, especially those comprising valsartan or a releasable form thereof, may be useful in the therapy (i.e. the treatment, prevention or delay of progression of) conditions and diseases associated with angiotensin II receptor activity, especially by receptor inhibition. Exemplary diseases and conditions include hypertension, congestive heart failure and myocardial infarction.

### pH modifier

As used herein the term "pH modifier" refers to an organic or inorganic chemical material that is able to release hydrogen ions (acid), for example an organic or inorganic acid, an acidic polymer, e.g. a carbomer, or a latent acid, and which is generally pharmaceutically acceptable. A pH modifier is employed in the invention to shift the microenvironmental pH of the acidic drug formulation to more acidic conditions in the less acidic parts of the intestine. The term "microenvironmental pH" as used herein includes reference to the pH within and in the vicinity of the drug formulation. The term "intestine" as used herein includes reference to the small intestine, including the duodenum, jejunum and ileum, and the large intestine, e.g. the colon. The composition may comprise one or more pH modifiers.

The pH modifier may be an acid having a pKa of from about 1 to about 7, in particular from about 2 to about 6.5, more particularly from about 3 to about 6.5. Of mention are pH modifiers having a pKa of 3.5 or less, for example less than 3.1, e.g. about 3 or less; these values are particularly preferred in the case of valsartan, the lowest pKa of which is 3.9. Where pKa values are mentioned herein, they are generally taken to be those as determined at a temperature of 25 °C in water. In some cases, the acidic strength of the pH modifier is at least the same as that of the acidic drug compound, i.e. it may have a pKa less than or equal to, especially less than, the pKa or, where two or more acidic groups are present, the most acidic pKa (referred to herein as "pKa1"), of the acidic drug compound.

The pH modifier may be an acid, in particular a latent acid, or a pharmaceutically acceptable salt thereof. Latent acids are compounds that hydrolyze to a free acid in presence of water, e.g., glucono-δ-lactone.

The use of solid acids or pharmaceutical acceptable salts thereof as pH modifiers is particularly convenient for the manufacture of compositions according to the invention.

In one embodiment of the invention, the pH modifier is an organic acid or a pharmaceutical acceptable salt thereof. Suitable organic acids contain one or more acidic groups, particularly compounds containing acidic groups selected from carboxylic and sulfonic acid groups, particularly those which are solid at ambient temperature, and especially those which have 2 or more acidic groups. In addition functional groups that modulate (e.g. amplify or diminish) the acidity of the acidic functional group may be present, for example hydroxyl-groups or amino-groups.

Particular water-soluble organic acids include a water- or poorly water-soluble organic acid selected from a mono, di- or polybasic carboxylic acid and a mono, di or tri-sulfonic acid, preferably those which are solid at ambient temperature. Particular solid water-soluble carboxylic acids include, for example aliphatic mono or poly-carboxylic acids such as those containing from 1 to 20 carbon atoms, particularly from 2 to 6 carbon atoms, more particularly all -or tricarboxylic acids containing from 4 to 6 and especially 4 carbon atoms, any of which acids may be saturated or unsaturated or having branched or non-branched carbon atom chains. Examples of suitable solid water-soluble aliphatic mono- carboxylic acids include sorbic acid (2,4-hexandienoic acid). Examples of suitable solid water-soluble aliphatic di-carboxylic acids include adipic, malonic, succinic, glutaric, maleic or fumaric acid. The aliphatic carboxylic acid may be optionally substituted by one or more groups (for example 1, 2 or 3), which may be the same or different, selected from e.g. carboxy, amino or hydroxy. Suitable substituted solid water-soluble aliphatic carboxylic acids include for example hydroxy substituted aliphatic mono-carboxylic acids such as gluconic acid, solid forms of lactic acid, glycolic acid or ascorbic acid; hydroxy substituted aliphatic di-carboxylic acids such as malic, tartaric, tartronic (hydroxymalonic), or mucic (galactaric) acid; hydroxy 2-substituted aliphatic tri-carboxylic acids, for example citric acid; or amino acids carrying an acidic side chain, such as glutamic acid or aspartic acid.

Suitable aromatic carboxylic acids include water-soluble aryl carboxylic acids containing up to 20 carbon atoms. Suitable aryl carboxylic acids comprise an aryl group, for example a phenyl or naphthyl group which carries one or more carboxyl groups (for example 1, 2 or 3 carboxy groups). The aryl group is optionally substituted by one or more groups (for example 1, 2 or 3), which may be the same or different selected from hydroxy, (1-4C) alkoxy (for example methoxy) and sulfonyl. Suitable examples of aryl carboxylic acids include, for example benzoic, phthalic, isophthalic, terephthalic or trimellitic acid (1,2,4- benzenetricarboxylic acid).

The pH modifier may comprise a polymeric organic acid or a pharmaceutically acceptable salt thereof. The polymer may be linear or branched or a combination thereof. The polymer may be cross-linked by one or more cross-linkers. Suitable polymeric acids include polymers containing a linear backbone with acidic groups, polymers containing a branched backbone with acidic groups, and mixtures thereof. Of particular mention are synthetic high-molecular-weight polymers of acrylic acid which may be crosslinked (e.g. Carbopol 71 G), or methacrylic acid polymers crosslinked with e.g. divinylbenzene (e.g. Amberlite IRP-64). Also of mention is alginic acid.

The pH modifier may comprise water-soluble in-organic acids include a water- or poorly water-soluble in-organic acids having a pKa in the above defined range for pH modifiers. An example of such in-organic acids are sodium-di-hydrogen-phosphate and -di-sodium-hydrogen-phosphate including all hydrates e.g. di-hydrate or mono-hydrate

Preferentially the pH modifier is selected from an organic acid, an acidic polymer, and a latent acid.

Especially where the acidic drug compound is valsartan or a pharmaceutically acceptable salt thereof, the pH modifier is selected from fumaric acid, aspartic acid, succinic acid, glutamic acid , adipic acid , succinic acid anhydride, cinnamic acid, ascorbic acid, ascorbyl palmitate, citric acid, sodium gutamine, malic acid, tartratic acid, L-lactic acid, maleic acid, oxalic acid, stearic acid, orotic acid, sebacic acid or, in each case, a pharmaceutically acceptable salt thereof including mixtures of two or more acids and/or salts. Especially preferred are fumaric acid and succinic acid. Fumaric acid has a pKa1 of about 3, more particularly of 3.03, at 25 °C, while succinic acid has a pKa1 of about 4.2.

The composition is therefore preferably adapted such that its microenvironmental pH is substantially maintained in at least a section of the Gl tract, especially one or more of the duodenum, jejunum, ileum and colon. In particular, the composition may be such that a microenvironmental pH of 5 or less (e.g. 4 or less, in particular 3.5 or less) is maintained in one or more of the duodenum, jejunum, ileum and colon. The quantity and properties of the pH modifier should therefore be tailored to optimise absorption of the acidic drug compound. This may involve the use of a pH modifier having a dissolution rate substantially similar to that of the acidic drug compound and/or a sufficient quantity of pH modifier to maintain the microenvironmental pH.

In a preferred embodiment of the present invention, the weight/weight ratio of pH-modifier to the acidic drug compound in the pharmaceutical composition is 0.01:1 or larger, preferably between 0.05:1 and 10:1, more preferably between 0.025:1 and 2:1. Of particular mention are compositions comprising valsartan in which the ratio of pH modifier to valsartan is from about 0.2:1 to about 1:0.3, in particular from about 0.25:1 to about 1:0.5, e.g. about 0.25:1, about 0.5:1, about 1:1 or about 1:0.5. Exemplary compositions comprise from about 5 to about 60 wt % pH modifier and from about 60 to about 5 wt % valsartan. Of particular mention are pH modifiers selected from fumaric acid and succinic acid.

For use in a sustained release composition, a pH modifier having a relatively poor water solubility, e.g. less than about 5% (g/100ml water), may be preferred, depending on the intended duration of action.

### Modified release forms

The composition may be adapted to provide modified (e.g. sustained or pulsatile) release of the acidic drug compound over a period of time, for example up to about 24 hours, e.g. up to about 16 or up to about 4 hours. The composition may be capable of maintaining blood levels of the drug within a relatively narrow range over a period of time. Similarly, the composition may also provide modified release of the pH modifier over the same period of time.

A particular type of modified release system is a diffusion-controlled system. Diffusion-controlled systems generally comprise a water-insoluble material which controls the ingress of water into and the subsequent egress of dissolved drug from the composition. Both diffusion and dissolution processes are involved in such systems. Examples of such diffusion controlled systems include gel-forming matrix tablets or coated tablets comprising a diffusion modifying film-coat. In addition, these systems may be eroding, whereby release of the drug and the pH modifier is at least partly controlled by this mechanism.

An example of a diffusion-controlled system is a matrix composition, in which the drug and pH modifier are dispersed throughout a matrix material. The matrix material binds at least the acidic drug compound and the pH modifier in such a way that the drug compound and/or pH modifier are released gradually over time in the Gl tract. Drug diffusion through the polymer matrix is usually the rate-limiting step, and the release rate is determined by the choice of matrix material and its consequent effect on the diffusion and partition coefficient of the drug to be released. Of particular mention are monolithic matrix systems, i.e. compositions which comprise a matrix containing a substantially uniform dispersion of the acidic drug compound and the pH modifier. Examples include matrix tablets and multiparticulate matrix systems, e.g. minitablets, granules or pellets. These systems may be eroding or non-eroding, and may be dispersable or dissolvable or show a mixture of this release mechanisms

The matrix material may comprise one or more matrix- or gel-forming polymers. For example, the composition may comprise one or more polymers selected from cellulose derivatives, acryl-derivatives, vinyl polymers, polyoxyethylene polymers, polyethylene glycol polymers and polysaccharides. Examples of acryl-derivatives include methacrylic acid co-polymers (e.g. Eudragit E30D, L100 or S100), aminoalkyl-methacrylate co-polymers (e.g. Eudragit RL100 or RS100), methacrylic ester co-polymers (e.g. Eudragit NE30D) and cross-linked acrylic acid polymers (e.g. Carbopol 71 G). Examples of vinyl polymers include polyvinylacetates (e.g. part of Kollidone SR), polyvinylacetate co-polymers (e.g. Kollidone VA64), polyvinylpyrrolidones (e.g. Kollidone K30) and its mixtures. Other suitable polymers include polyoxyethylene (e.g. Polyox WSR31 0 manufactured by Dow Chemicals, USA), polyethylene glycols (e.g. PEG with a molecular weight of more than 4000), polysaccharides e.g. (xanthans, xanthan gum, galactomannan, pectin and alginates). A preferred polymer is hydroxypropyl methyl cellulose.

The polymers may, for example, comprise different polymer chain lengths, different substitution ratios of polymer backbone to functional group, or, if more than one functional group is present, different ratios of functional groups. An example of the latter case is Hypromelose 2208 (Methocel K100LV CR, manufactured by Dow Chemicals), which has a methoxyl content of 19-24% corresponding to an average substitution ratio of 1.4 and a hydroxypropyl content of 7-12% corresponding to an average substitution ratio of 0.21 of the maximum 3 hydroxyl-groups in the glucose backbone on this cellulose polymer.
Furthermore, polymers may be used in different particle sizes, e.g. cellulose derivatives can be manufactured in different particle size distributions. An example is Methocel K100LC, which can be obtained in fine or coarse form.

The matrix material may comprise cellulose derivatives comprising linkage groups from the polymer backbone to functional groups that are ether, ester or a combination of ether and ester on the same cellulose backbone polymer. Examples include alkyl-ether celluloses e.g. methyl- or ethyl-cellulose, hydroxyl-alkyl-ether celluloses e.g. hydroxyethyl- or hydroxypropylcellulose, mixed alkyl and hydroxyl-alkyl celluloses e.g. hydroxypropyl methyl- or hydroxyethyl-methyl cellulose, carbon-acid-ester celluloses e.g. acetyl-cellulose, mixed ether and ester celluloses, e.g. sodium carboxy-methyl- cellulose or hydroxypropyl-methyl cellulose-phthatlate or cellulose-acetate-phthalate or cellulose acetate propionate or cellulose acetate butyrate or cellulose butyrate or cellulose nitrate or carboxymethly-ethoxyl cellulose or hydroxypropyl methyl cellulose acetate succinate.

Another type of diffusion-controlled system comprises a core (inner "layer") and an outer layer. In one embodiment, the core contains the pH modifier and the outer layer contains the drug compound. In another embodiment, the core contains the drug compound and the outer layer contains the pH modifier. In a further embodiment, the core contains both the pH modifier and the drug compound. The core, the outer layer or both can contain one or more water-insoluble polymers that control the release of the drug and/or the pH-modifier. An example of such a system is a press-coated tablet.

A composition of the invention may comprise a diffusion coating which controls the ingress of water and egress of dissolved drug from the composition. A diffusion coating therefore is usually permeable, at least in part, to water. The composition may comprise a core containing the drug compound and pH modifier, and a diffusion coating which modulates release of the acidic drug compound and/or pH modifier. In addition the core may also comprise one or more water-insoluble polymers that control the release of the drug and/or the pH-modifier. Examples of such systems include coated tablets, e.g. monolithic matrix tablets, and coated multiparticulate systems, e.g. minitablets, pellets, granules and beads. When the composition is in the form of a tablet, it is preferably a tablet which is able to disintegrate or dissolve in the mouth, stomach or small intestine to give modified release coated multiparticles. Compared to monolithic systems, multiparticulates display the advantage that the mean gastric emptying is faster and less dependent on the nutritional state as they are sufficiently small to be evacuated through the pylorus. Multiparticulates can have numerous formulation applications. For example, they may be filled in a capsule shell or as a sachet or they may be compressed into a tablet. Drug release can be supported by additional excipients such as osmotic active materials, e.g. NaCl, or surfactants, e.g. SDS or Tween compounds.

The diffusion coating may be porous to allow water to ingress into the composition. Alternatively or additionally, the diffusion coating may contain a pore-forming agent, i.e. an agent which is capable of forming one or more pores in the coating during use. A diffusion coating may comprise a water-insoluble material and a water-soluble pore-forming agent. When the coating is contacted with water, the pore-forming agent will dissolve thereby forming one or more pores in the coating. Examples of water-insoluble materials include polymers based on one or more of ethylcellulose, acrylic acid ester and methacrylic acid ester. Examples of pore-forming agents include water-soluble polymer such as hydroxypropylmethylcellulose and polyethyleneglycol.

The composition may comprise a mixture of immediate release and modified release monolithic solid dosage forms or multiparticulates, which may be filled in a capsule shell or present in a sachet. The overall release of drug from such system on administration of the dosage form is characterized by a rapid release of drug followed by a modified (e.g. sustained or pulsed) release of the drug over time.

### Other components

In certain exemplary embodiments of the present invention, the pharmaceutical composition may comprise additional excipients commonly found in pharmaceutical compositions, examples of such excipients include, but are not limited to, fillers, glidants, lubricants, binders, antioxidants, antimicrobial agents, enzyme inhibitors or substrates, stabilizers, preservatives, flavors, sweeteners and other components as e.g. described in Handbook of Pharmaceutical Excipients, Rowe et al., Eds., 4th Edition, Pharmaceutical Press (2003), which is hereby incorporated by reference.

Additional excipients with the exception of fillers and/or binders may comprise from about 0.05-11 % by weight of the total pharmaceutical composition, e.g. from about 0.5 to about 3.5% by weight of the total composition. Antioxidants, anti-microbial agents, enzyme inhibitors or substrates, stabilizers or preservatives typically provide up to about 0.05-10% by weight of the total pharmaceutical composition. Sweetening or flavoring agents typically provide up to about 2.5% or 5% by weight of the total pharmaceutical composition. Lubricants typically provide up to about 10%, preferentially about 1%, by weight of the total pharmaceutical composition.

Examples of a "lubricant", as used herein, include, but are not limited to magnesium stearate, talc, hydrogenated castor oil, glycerylbehaptate, glycerolmonostearate, polyethylene glycol, ethylene oxide polymers, sodium lauryl sulfate, magnesium lauryl sulfate, sodium oleate, sodium stearyl fumarate, DL-leucine, collidal silica, and others known in the art.

Examples of a "filler", as used herein, include, but are not limited to lactose (which may be in an anhydrous or hydrated form), sugar, starches (for example corn, wheat, maize, potato), modified starches (e.g., starch hydrolysates or pregelatinized starch), mannitol, sorbitol, trehalose, maltose, glucose anhydrate; inorganic salts (e.g., calcium carbonate, magnesium carbonate, dibasic calcium phosphate, tribasic phosphate, calcium sulfate), microcrystalline cellulose, cellulose derivates.

Examples of a "glidant" as used herein, include, but are not limited to silica aerogels like Aerosil 200 or talc.

Examples of a "binder" as used herein, include, but are not limited to hydroxypropylmethylcellulose (HPMC), e.g. HMPC with a low apparent viscosity, e.g. below 100 cps as measured at 20°C for a 2 % by weight aqueous solution, e.g. below 50 cps, preferably below 20 cps, for example HPMC 3 cps, as known and commercially available under the name Pharmacoat® 603 from the Shin-Etsu company, other suitable binders for a composition of the present are polyvinylpyrrolidone (PVP), e.g. PVP K30 or PVP K12, as known and commercially available under the trade name Povidone® from the BASF company.

Examples of antioxidants include, but are not limited to, ascorbic acid and its derivatives, tocopherol and its derivatives, butyl hydroxyl anisole and butyl hydroxyl toluene. Vitamin E as α-tocopherol is particularly useful.

The compositions of the invention may comprise an enteric coating, to prevent drug and/or acid dissolution from the solid dosage form before reaching the small intestine. A subcoat may be applied in order to separate the enteric coating from a matrix comprising the pH modifier.

By way of example, an enteric coating (% of final weight) may contain:
- 2-40 % polymers for enteric coating, for example hydroxypropylmethylcellulose phthalate (e.g. HP 50, HP 55 from Shin Etsu), hydroxypropylmethylcellulose acetate succinate (e.g. Aqoat types H, M, L from Shin Etsu), methyl acrylic acid-ethyl acrylic acid copolymer (methacrylic acid copolymer, USP) (e.g. Eudragit L, S, L100-55, L30D from Röhm Pharma, Acryl-Eze from Colorcon, Kollicoat MAE 30 DP from BASF), celluloseacetatephthalate (e.g. Aquacoat CPD from FMC Biopolymer, or Polymer from Eastman Kodak), polyvinylacetatephthalate (Sureteric, Colorcon);
- 0-15 % polymers for subcoating: hydroxypropylmethylcellulose (Pharmacoat 603 or 606), ethylcellulose (i.e. Aquacoat ECD, FMC Biopolymer, Surelease, Colorcon) and or mixtures thereof with a ratio of ethylcellulose:HPMC of 1:1 up to 1:10), polyvinylalcohol (Opadry II HP, type 85F, Colorcon);
- 0-50 % plasticizers (triacetine, triethylcitrate, PEG 4000, PEG 6000, PEG 8000, diethylphthalate, diethylsebacate, acetyltriethylcitrate etc.);
- 0-15 % antisticking agents (e.g. Aerosil 200, Syloid 244 FP, talcum, glycerol monostearate etc.);
- organic solvents or mixtures thereof with and without parts of water (ethanol, acetone, isopropanol) or water q.s. to dissolve or disperse the coating polymers and excipients for coating solution; and
- 0-0.5 % sodium hydroxide for redispersion of polymers for aqueous enteric coating suspensions (e.g. Eudragit L100-55).

The following examples are illustrative, but do not serve to limit the scope of the invention described herein. The examples are meant only to suggest a method of practicing the present invention. Quantities of ingredients, represented by percentage by weight of the pharmaceutical composition, used in each example are set forth in the respective tables located after the respective descriptions.

### EXAMPLES

### 1. Monolithic matrix modified release systems

### 1.1 Formulation ingredients & ranges

- 0.01-80% active ingredient
- 1-70% pH modifier (e.g. an organic acid such as citric acid, fumaric acid, succinic acid, adipic acid, maleic acid)
- 3-60% polymer (e.g. Methocel K100M, Methocel K100LV, Klucel HF, Metholose 60SR, or mixtures thereof)
and optionally one or more of:
- 0.1-2% of a glidant e.g. Aerosil 200
- 0.1-2% of a lubricant e.g. magnesium stearate
- additional tabletting excipients such as fillers (e.g. lactose) or binders

### 1.2 Preparation of granules for tablets and minitablets

Granules are prepared by a wet granulation or roller compaction process.

### 1.2.1 Preparation of granules by wet granulation

The active ingredient, the organic acid, the polymer and any additional tabletting excipients are mixed and wet granulated by water or organic solvents. The dried granules for the preparation of the monolithic matrix tablets are sieved through a suitable sieve, e.g. having openings of 1.0 mm diameter. In the case of the minitablets granules are sieved through a suitable sieve with smaller openings, e.g. 0.4 mm. An outer phase consisting of Aerosil, magnesium stearate and any additional tabletting excipients is added and mixed thoroughly. The blend is compressed into monolithic matrix tablets of a diameter of 5 to 12 mm or minitablets of a diameter of e.g. 1.7 to 2 mm.

### 1.2.2 Preparation of granules by roller compaction

The active ingredient, the organic acid, the polymer and any additional tabletting excipients are mixed and subsequently compacted using a roller compactor. The ribbons formed by the roller compactor are milled for the preparation of the monolithic matrix tablets through a suitable sieve, e.g. 1 mm. In the case of the minitablets, the ribbons are milled using a sieve with smaller openings, e.g. 0.4 mm. An outer phase consisting out of Aerosil, magnesium stearate and any additional tabletting excipients is added and mixed thoroughly. The blend is compressed into monolithic matrix tablets having a diameter of from 5 to 12 mm or minitablets having a diameter of, for example, from 1.7 to 2 mm.

### 1.3 Composition of tablet cores for matrix minitablets

The following minitablet compositions are prepared by wet granulation and have a weight of 250±5 mg (∅10 mm). 250 mg±5 mg of the prepared minitablets is filled into capsules.

In each case, the drug substance is either valsartan (Diovan) acid or its calcium salt, e.g. the tetrahydrate form of the calcium salt (calculated as active moiety).

| **Example 1** | | | | **Example 2** | | |
|---|---|---|---|---|---|---|
| | [%] | mg/tablet/ capsule | | | [%] | mg/tablet/ capsule |
| Methocel K100LV | 30.00 | 75.00 | | Methocel K100LV | 30.00 | 75.00 |
| Diovan acid or Ca | **10.00** | **25.00** | | Diovan acid or Ca | **10.00** | **25.00** |
| Fumaric acid | **20.00** | **50.00** | | Fumaric acid | **0.00** | **0.00** |
| Lactose milled | 34.80 | 87.00 | | Lactose milled | 54.80 | 137.00 |
| HPMC 3cps | 2.67 | 6.68 | | HPMC 3cps | 2.67 | 6.68 |
| Mg-Stearat | 1.00 | 2.50 | | Mg-Stearat | 1.00 | 2.50 |
| Aerosil | 1.53 | 3.83 | | Aerosil | 1.53 | 3.83 |
| | 100.00 | 250.00 | | | 100.00 | 250.00 |
| | | | | | | |
| **Example 3** | | | | **Example 4** | | |
| | [%] | mg/tablet/ capsule | | | [%] | mg/tablet/ capsule |
| Methocel K100LV | 30.00 | 75.00 | | Methocel K100LV | 30.00 | 75.00 |
| Diovan acid or Ca | **20.00** | **50.00** | | Diovan acid or Ca | **20.00** | **50.00** |
| Fumaric acid | **20.00** | **50.00** | | Fumaric acid | **0.00** | **0.00** |
| Lactose milled | 24.80 | 62.00 | | Lactose milled | 44.80 | 112.00 |
| HPMC 3cps | 2.67 | 6.68 | | HPMC 3cps | 2.67 | 6.68 |
| Mg-Stearat | 1.00 | 2.50 | | Mg-Stearat | 1.00 | 2.50 |
| Aerosil | 1.53 | 3.83 | | Aerosil | 1.53 | 3.83 |
| | 100.00 | 250.00 | | | 100.00 | 250.00 |
| | | | | | | |

| **Example 5** | | | | **Example 6** | | |
|---|---|---|---|---|---|---|
| | [%] | mg/tablet/ capsule | | | [%] | mg/tablet/ capsule |
| Methocel K4M | 30.00 | 75.00 | | Methocel K4M | 30.00 | 75.00 |
| Diovan acid or Ca | **10.00** | **25.00** | | Diovan acid or Ca | **10.00** | **25.00** |
| Fumaric acid | **20.00** | **50.00** | | Fumaric acid | **0.00** | **0.00** |
| Lactose milled_ | 34.00 | 85.00 | | Lactose milled | 54.00 | 135.00 |
| HPMC 3cps | 2.67 | 6.68 | | HPMC 3cps | 2.67 | 6.68 |
| Mg-Stearat | 1.33 | 3.33 | | Mg-Stearat | 1.33 | 3.33 |
| Aerosil | 2.00 | 5.00 | | Aerosil | 2.00 | 5.00 |
| | 100.00 | 250.00 | | | 100.00 | 250.00 |
| | | | | | | |

| **Example 7** | | | | **Example 8** | | |
|---|---|---|---|---|---|---|
| | [%] | mg/tablet/ capsule | | | [%] | mg/tablet/ capsule |
| Methocel K100LV | 30.00 | 75.00 | | Methocel K100LV | 30.00 | 75.00 |
| Lactose milled | 34.00 | 85.00 | | Diovan acid or Ca | **30.00** | **75.00** |
| Diovan acid or Ca | **30.00** | **75.00** | | Fumaric acid | **15.00** | **37.50** |
| Fumaric acid | **0.00** | **0.00** | | Lactose milled | 19.00 | 47.50 |
| HPMC 3cps | 2.67 | 6.68 | | HPMC 3cps | 2.67 | 6.68 |
| Mg-Stearat | 1.33 | 3.33 | | Mg-Stearat | 1.33 | 3.33 |
| Aerosil | 2.00 | 5.00 | | Aerosil | 2.00 | 5.00 |
| | 100.00 | 250.00 | | | 100.00 | 250.00 |

| **Example 9** | | | | | | |
|---|---|---|---|---|---|---|
| | [%] | mg/tablet/ capsule | | | | |
| Methocel K100LV | 30.00 | 75.00 | | | | |
| Lactose milled | 4.00 | 10.00 | | | | |
| Diovan acid or Ca | **30.00** | **75.00** | | | | |
| Fumaric acid | **30.00** | **75.00** | | | | |
| HPMC 3cps | 2.67 | 6.68 | | | | |
| Mg-Stearat | 1.33 | 3.33 | | | | |
| Aerosil | 2.00 | 5.00 | | | | |
| | 100.00 | 250.00 | | | | |

### 1.4 Composition of tablet cores for matrix tablets

Matrix tablets having the following compositions are prepared. The tablet of Example 10 is prepared by wet granulation, while the tablets of Examples 11 to 20 are prepared by roller compaction.

In each case, the drug substance is either valsartan (Diovan) acid or its calcium salt, e.g. the tetrahydrate form of the calcium salt (calculated as active moiety).

| **Example 10** | % | mg/tablet | | **Example 11** | % | mg/tablet |
|---|---|---|---|---|---|---|
| Inner Phase I | | | | Inner Phase I | | |
| Valsartan Ca | 30 | 200.32 | | Valsartan Ca | 30 | 200.10 |
| Methocel K100LV | 30 | 200.00 | | Methocel K100LV | 30 | 200.10 |
| Fumaric acid | 30 | 200.10 | | Fumaric acid | 30 | 200.10 |
| Lactose spray dried | 8 | 53.18 | | Lactose spray dried | 6.5 | 43.36 |
| Water | | qs | | Aerosil 200 | 1 | 6.67 |
| Outer Phase II | | | | Mg-stearat | 0.5 | 3.34 |
| Aerosil 200 | 1 | 6.70 | | Outer Phase II | | |
| Mg-stearat | 1 | 6.70 | | Aerosil 200 | 1 | 6.67 |
| **Total** | 100 | 667.00 | | Mg-stearat | 1 | 6.67 |
| | | | | **Total** | 100 | 667.01 |
| | | | | | | |

| **Example 12** | % | mg/tablet | | **Example 13** | % | **mg/tablet** |
|---|---|---|---|---|---|---|
| Inner Phase I | | | | Inner Phase I | | |
| Valsartan Ca | 30 | 200.10 | | Valsartan free acid | 30 | 160.00 |
| Methocel K100LV | 30 | 200.10 | | Methocel K100LV | 30 | 160.00 |
| Lactose spray dried | 36.5 | 243.46 | | Fumaric acid | 30 | 160.00 34.50 |
| Aerosil 200 | 1 | 6.67 | | Lactose spray dried | 6.5 | |
| Mg-stearat | 0.5 | 3.34 | | Aerosil 200 | 1 | 5.30 |
| Outer Phase II | | | | Mg-stearat | 0.5 | 2.60 |
| Aerosil 200 | 1 | 6.67 | | Outer Phase II | | |
| Mg-stearat | 1 | 6.67 | | Aerosil 200 | 1 | 5.30 |
| **Total** | 100 | 667.01 | | Mg-stearat | 1 | 5.30 |
| | | | | **Total** | 100 | 533.00 |
| | | | | | | |

| **Example 14** | % | mg/tablet | | **Example 15** | % | mg/tablet |
|---|---|---|---|---|---|---|
| Inner Phase I | | | | Inner Phase I | | |
| Valsartan free acid | 30 | 160.00 | | Valsartan free acid | 30 | 80.00 |
| Methocel K100LV | 30 | 160.00 | | Methocel K100LV | 30 | 80.00 |
| Lactose spray dried | 36.5 | 194.50 | | Lactose spray dried | 36.5 | 97.25 |
| Aerosil 200 | 1 | 5.30 | | Aerosil 200 | 1 | 2.65 |
| Mg-stearat | 0.5 | 2.60 | | Mg-stearat | 0.5 | 1.30 |
| Outer Phase II | | | | Outer Phase II | | |
| Aerosil 200 | 1 | 5.30 | | Aerosil 200 | 1 | 2.65 |
| Mg-stearat | 1 | 5.30 | | Mg-stearat | 1 | 2.65 |
| **Total** | 100 | 533.00 | | **Total** | 100 | 266.50 |
| | | | | | | |

| **Example 16** | % | mg/tablet | | **Example 17** | % | mg/tablet |
|---|---|---|---|---|---|---|
| Inner Phase I | | | | Inner Phase I | | |
| Valsartan Ca | 40.02 | 200.10 | | Valsartan Ca | 40.02 | 200.10 |
| Methocel K100LV | 30.00 | 150.00 | | Methocel K100LV | 30.00 | 150.00 |
| Fumaric acid | 10.00 | 50.00 | | Fumaric acid | 10.00 | 50.00 |
| Lactose spray dried | 15.28 | 76.40 | | Lactose spray dried | 15.28 | 76.40 |
| Aerosil 200 | 1.00 | 5.00 | | Aerosil 200 | 1.00 | 5.00 |
| Mg-stearat | 1.70 | 8.50 | | Mg-stearat | 1.70 | 8.50 |
| Outer Phase II | | | | Outer Phase II | | |
| Aerosil 200 | 1.00 | 5.00 | | Aerosil 200 | 1.00 | 5.00 |
| Mg-stearat | 1.00 | 5.00 | | Mg-stearat | 1.00 | 5.00 |
| **Total** | 100 | 500.00 | | **Total** | 100 | 500.00 |
| | | | | | | |

| **Example 18** | % | mg/tablet | | **Example 19** | % | mg/tablet |
|---|---|---|---|---|---|---|
| Inner Phase I | | | | Inner Phase I | | |
| Valsartan Ca | 40.02 | 200.10 | | Valsartan Ca | 40.02 | 200.10 |
| Metolose 60SH-50 | 30.00 | 150.00 | | Methocel K100LV | 30.00 | 150.00 |
| Fumaric acid | 10.00 | 50.00 | | Succinic acid | 10.00 | 50.00 |
| Lactose spray dried | 15.28 | 76.40 | | Lactose spray dried | 15.28 | 76.40 |
| Aerosi1200 | 1.00 | 5.00 | | Aerosil 200 | 1.00 | 5.00 |
| Mg-stearat | 1.70 | 8.50 | | Mg-stearat | 1.70 | 8.50 |
| Outer Phase II | | | | Outer Phase II | | |
| Aerosil 200 | 1.00 | 5.00 | | Aerosil 200 | 1.00 | 5.00 |
| Mg-stearat | 1.00 | 5.00 | | Mg-stearat | 1.00 | 5.00 |
| **Total** | 100 | 500.00 | | **Total** | 100.00 | 500.00 |
| | | | | | | |
| | | | | | | |

| **Example 20** | % | mg/tablet | | | | |
|---|---|---|---|---|---|---|
| Inner Phase I | | | | | | |
| Valsartan Ca | 40.02 | 200.10 | | | | |
| Metolose 60SH-50 | 30.00 | 150.00 | | | | |
| Succinic acid | 10.00 | 50.00 | | | | |
| Lactose spray dried | 15.28 | 76.40 | | | | |
| Aerosil 200 | 1.00 | 5.00 | | | | |
| Mg-stearat | 1.70 | 8.50 | | | | |
| Outer Phase II | | | | | | |
| Aerosil 200 | 1.00 | 5.00 | | | | |
| Mg-stearat | 1.00 | 5.00 | | | | |
| **Total** | 100.00 | 500.00 | | | | |

### 1.5 Compression-coated tablets comprising a pH modifier

For the preparation of the inner core tablet comprising the acid, tablets were made from pure acid mixed with a lubricant, e.g. from pure succinic acid, fed manually into the die of a single-punch tabletting machine (EK0, Korsch, Germany). In another embodiment, the inner core tablet was compressed from granules made of acid and a filler, water-soluble or water-insoluble, preferably water-insoluble, mixed with a lubricant. The matrix granules for the outer layer were prepared according to the method described above (1.2. and 1.4), but could also comprise only drug and polymer without any acid.

For the compression-coated tablet, the core tablet was placed in the center of the outer layer (e.g. the granules of the outer layer were filled into the die to make a powder bed, on the center of which the core tablet was placed before being covered by further granules of the outer layer) and a compession force was being applied.

Composition of a compression-coated tablet made from matrix granules comprising an inner core of succinic acid and an outer layer comprising succinic acid:

### Example 21

| | **Composition** | **Weight** **(mg)** | **%** | **%** |
|---|---|---|---|---|
| **Outer layer** | Ca-Valsartan (160mg) | 200 | 37.3 | 25.5 |
| | Methocel K100LV | 200 | 37.3 | 25.5 |
| | Lactose monohydrate | 38 | 7.1 | 4.8 |
| | succinic acid | 85 | 15.9 | 10.8 |
| | Magnesium stearate | 7.5 | 1.4 | 1.0 |
| | Aerosil | 5 | 0.9 | 0.6 |
| | | 535.5 | 100.0 | |
| **Inner core** | Succinic acid | 250 | | 31.8 |
| **Total** | | 785.5 | | 100.0 |

### 1.6 Preparation of enteric coating and subcoating

The above described matrix minitablets and matrix tablets may be coated with an enteric coating.

An isolation coat is applied from an aqueous solution of HPMC (4-8%), plasticizer (0-3%) and antisticking agents (0-3%). Aquacoat ECD or Surelease (aqueous ethylcellulose dispersion) might be added in the range of 1:10 up to 1:1 (Ethylcellulose:HPMC) to improve the isolation effect of the subcoating. Based on the tablet size, the total amount of sub coat applied is between 3-15% (more probably 5-10%). Polyvinylalcohol (Opadry II HP) in a range of 2-10% of core weight can be employed for an effective subcoating. Furthermore, a HPMC subcoat may be coated from organic suspension in ethanol/acetone 1:1 (about 6-10% polymer per solvent) without any further additives.

In the case of an organic enteric coating solution, after dissolving the enteric coating polymer and the plasticizer in organic solvents, the antisticking agents are dispersed. With regard to coating from aqueous dispersions, the plasticizer is dissolved or finely dispersed in water, the antisticking agent is dispersed, and finally the reconstituted suspension (i.e. Aqoat or Eudragit L 100-55,) or the commercially available aqueous polymer dispersion (Eudragit L 30D, Acryl-Aze, Kollicoat MAE 30 D) are added.

The coating is applied using a pan coater or fluidized bed coater with or without Wurster principle up to a coating layer between 2 and 35 % (more preferred about 5-10% for large tablets and 10-20% for small tablets/minitablets) at a product temperature between 28 and 50°C . The layer depends on the tablet size to assure an enteric resistance for 1-3 hours in artificial gastric juice or 0.1 N HCL solution (acc. to Ph Eur. or USP). Additionally, swelling of the tablet core during gastric resistance test should be reduced to a minimum.

| **Example Coating A** | parts | % | mg/250 mg core | mg/8 mg core |
|---|---|---|---|---|
| Sub coat | | | | |
| HPMC 3 cps | 5 | 25 | 12.5 | 0.8 |
| Triethylcitrate | 0.5 | 2.5 | 1.25 | 0.08 |
| Talc | 0.5 | 2.5 | 1.25 | 0.08 |
| Water q.s. | | | | |

| Enteric coat | | | | |
|---|---|---|---|---|
| Eudragit L 30 D (dry) | 10 | 50 | 25 | 1.6 |
| PEG 6000 | 2 | 10 | 5 | 0.32 |
| Syloid 244 FP | 2 | 10 | 5 | 0.32 |
| Water q.s | | | | |
| Total (dry) | 20 | 100 | 50 | 3.2 |

| **Example Coating B** | parts | % | mg/250 mg core | mg/8 mg core |
|---|---|---|---|---|
| Sub coat | | | | |
| HPMC 3 cps | 6 | 26.67 | 15.0 | 0.96 |
| Aquacoat ECD (dry) | 2 | 8.89 | 5.0 | 0.32 |
| Triethylcitrate | 0.6 | 2.67 | 1.5 | 0.096 |
| Glycerol monostearate | 0.4 | 1.77 | 1.0 | 0.064 |
| Water q.s. | | | | |

| Enteric coat | | | | |
|---|---|---|---|---|
| HPMC AS (Aqoat) MF | 10 | 44.44 | 25.0 | 1.60 |
| Triethylcitrate | 2.5 | 11.11 | 6.25 | 0.40 |
| Talc | 1 | 4.44 | 2.5 | 0.16 |
| Water q.s. | | | | |
| Total (dry) | 22.5 | 100.0 | 56.25 | 3.60 |
| | | | | |

| **Example Coating C** | parts | % | mg/250 mg core | mg/8 mg core |
|---|---|---|---|---|
| Sub coat | | | | |
| HPMC 3 cps | 5 | 32.5 | 12.5 | 0.8 |
| Ethanol/Acetone 1.1: q.s. | | | | |
| Enteric coat | | | | |
| HP 50 | 8 | 51.9 | 20.0 | 1.28 |
| Triacetine | 0.8 | 5.2 | 2.0 | 0.13 |
| Aerosil 200 | 1.6 | 10.4 | 4.0 | 0.26 |
| Ethanol/Acetone 1:1 : q.s. | | | | |
| Total (dry) | 15.4 | 100.0 | 38.5 | 2.47 |
| | | | | |
| **Example Coating D** | parts | % | mg/250 mg core | mg/8 mg core |
| Sub coat | | | | |
| Opadry II HP | 4 | 21.46 | 10 | 0.64 |
| Water q.s. | | | | |

| Enteric coat | | | | |
|---|---|---|---|---|
| Eudragit L100-55 | 10 | 53.65 | 25 | 1.6 |
| Sodium hydroxide | 0.14 | 0.75 | 0.35 | 0.022 |
| Triethylcitrate | 2.5 | 13.41 | 6.25 | 0.4 |
| Syloid 244 FP | 2 | 10.73 | 5.0 | 0.32 |
| Water q.s | | | | |
| Total dry | 18.64 | 100.0 | 46.6 | 2.982 |
| | | | | |

| **Example Coating E** | parts | % | mg/250 mg core | mg/8 mg core |
|---|---|---|---|---|
| Enteric coat | | | | |
| HP 50 | 10 | 71.43 | 25 | 1.6 |
| Diethylsebacate | 1 | 7.14 | 2.5 | 0.16 |
| Talc | 3 | 21.43 | 7.5 | 0.48 |
| Ethanol/Acetone 1:1 q.s. | | | | |
| Total dry | 14 | 100.0 | 34 | 2.24 |
| | | | | |

| **Example Coating F** | parts | % | mg/250 mg core | mg/8 mg core |
|---|---|---|---|---|
| Enteric coat | | | | |
| Eudragit L 100-55 | 10 | 76.92 | 25 | 1.6 |
| Triethylcitrate | 1 | 7.69 | 2.5 | 0.16 |
| Syloid 244 FP | 2 | 15.38 | 5.0 | 0.32 |
| Isopropanol/Water 97.3: q.s | | | | |
| Total dry | 13 | 100.0 | 32.5 | 2.08 |

### 2. Diffusion coated modified release systems

### 2.1 Preparation of multiparticulate systems

### 2.1.1 Preparation of minitablets

The active ingredient, pH modifier and any additional tabletting excipients are mixed and wet granulated using water or organic solvents. The dried granules are, for example, sieved through a 400 µm sieve and compressed in minitablets. For compression purposes, an outer phase consisting out of Aerosil and magnesium stearate is added and mixed thoroughly. The blend is compressed into minitablets of a diameter of, for example, from 1.25 to 4 mm, in particular from 1.7 to 2 mm. The resulting minitablets are finally coated with one of the coating formulations using polymers as described below (i.e. diffusion coat, diffusion coat with an additional enteric coat, diffusion coat comprising an enteric polymer).

An exemplary minitablet formulation has the following composition:

| **Example 22** | % | mg/250mg tablets |
|---|---|---|
| Lactose anhydrous | 24.14 | 60.35 |
| Fumaric acid | 20.00 | 50.00 |
| Diovan acid or Ca | 20.00 | 50.00 |
| Avicel PH 102 | 33.33 | 83.33 |
| Aerosil 200 | 1.53 | 3.83 |
| Mg-stearat | 1.00 | 2.50 |
| | 100.00 | 250.00 |

### 2.1.2 Preparation of pellets

In one embodiment, a dry blend is made by mixing the drug, microcrystalline cellulose (i.e. Avicel PH101) and lactose in a planetary mixer. Purified water is added to give a wet mass that is subsequently extruded using a screen of a suitable size. The extrudates are rounded in a spheroniser, thoroughly dried and sieved for suitable size selection. Finally, the resulting pellets are coated with an aqueous solution or aqueous dispersion of coating formulations below (i.e. diffusion coat, diffusion coat with an additional enteric coat, diffusion coat comprising an enteric polymer).

Exemplary pellet formulations have the following composition (amounts given in %):

| **Example 23** | [%] | | **Example 24** | [%] |
|---|---|---|---|---|
| Diovan acid or Ca (calculated as active moiety) | 20% | | Diovan acid or Ca (calculated as active moiety) | 30% |
| Fumaric acid | 20 % | | Fumaric acid | 20% |
| Lactose (standard quality) | 10% | | Lactose (standard quality) | 8% |
| Microcrystalline cellulose (Avicel PH101) | 50% | | Microcrystalline cellulose (Avicel PH101) | 42% |
| Water for wet massing | q.s.* | | Water for wet massing | q.s.* |
| | | | | |
| **Example 25** | [%] | | | |
| Diovan acid or Ca (calculated as active moiety) | 10% | | | |
| Fumaric acid | 20% | | | |
| Lactose (standard quality) | 15% | | | |
| Microcrystalline cellulose (Avicel PH101) | 55% | | | |
| Water for wet massing | q.s*. | | | |

| | | | | |
|---|---|---|---|---|
| * removed during processing. | | | | |

### 2.2 Diffusion coating compositions

### 2.2.1 Diffusion coating ingredients & ranges

Exemplary coating ingredients and ranges are as follows:
- 1-20 % polymers for diffusion coating, e.g. ethylcellulose (Aquacoat ECD, FMC Biopolymer, Surrelease, Colorcon), acrylic/methacrylic acid-ester/Eudragit RL, Eudragit RS (Röhm);
- 0-20 % water soluble polymers as pore formers, e.g. hydroxypropylmethylcellulose 3, 6 cps (Pharmacoat 603, 606, Shin-Etsu), polyethylenglycol (PEG 2000 - PEG 8000);
- 0-15 % polymers for subcoating, for example hydroxypropylmethylcellulose (Pharmacoat 603 or 606), ethylcellulose (e.g. Aquacoat ECD, FMC Biopolymer, Surelease, Colorcon) and or mixtures thereof with a ratio of ethylcellulose:HPMC = 1:1 up to 1:10), Polyvinylalcohol (Opadry II HP, type 85F, Colorcon);
- 0-20 % enteric coating polymers as pore formers (e.g. see the list above);
- 0-10 % plasticizers (triacetine, triethylcitrate, PEG 4000, PEG 6000, PEG 8000, Diethyl phthalate, Diethylsebacate, Dibuthylsebacate, Acetyltriethylcitrate etc.);
- 0-15 % antisticking agents (Aerosil 200, Syloid 244 FP, Talcum, Glycerol monostearate etc.); and
- organic solvents or mixtures thereof with and without parts of water (ethanol, acetone, isopropanol) or water q.s. to dissolve or disperse the coating polymers and excipients for coating solution.

In the case of an organic solution, the plasticizer and polymers are dissolved in the organic solvent mixture and finally the antisticking agent is dispersed. For an aqueous dispersion, the plasticizer is dissolved in water and the antisticking agent is finely dispersed using a homogenizer. Finally the pre-prepared polymer dispersion (as commercially available) or predispersed in water is added to the plasticizer/antisticking agent/water mixture and stirred for some time before spraying.

### 2.2.2 Diffusion coatings based on acrylic/methacrylic acid ester polymers

The polymers used for diffusion coating are Eudragit RS /RL mixtures in a ratio of 1:1 up to 9:1 from aqueous suspension or organic solution. Suitable plasticizers are triethylcitrate, dibutylsebacate, Triacetine in a range of 1 to 30% of coating dispersion (5-20%). Eudragit RS could be combined with the enteric coating polymer as pore former like hydroxypropylmethylcellulose acetate succinate, Type Aqoat type M(MF) or H (HF) in organic solution or aqueous dispersion or with hydroxypropylmethylcellulose phthalate (i.e. HP 50, HP 55) in organic solution. An enteric pore former suppresses the drug release in the acidic environment in the stomach. After solution of the enteric pore former in intestinal juice with pH > 5.5 the drug will dissolve and uniformly owing to the low microenvironmental pH inside the solid dosage form. Thereby, less inter- and intra subject variance is expected.

The coating layer is applied between 5 and 40%, usually between 7 and 15 %, i.e. 10% of core weight. The ratio of Eudragit RS and enteric pore former may be varied between 95:5 up to 50:50 to adapt the release profile.

| **Example Coating G** | parts | % (dry) | % (liquid) | mg/8 mg core |
|---|---|---|---|---|
| Eudragit RL 30 D | 1.52 | 6.9 | 4.62 | 0.06 |
| Eudragit RS 30D | 13.76 | 62 | 41.70 | 0.50 |
| Triethylcitrate | 2.8 | 12.5 | 2.80 | 0.10 |
| Syloid 244 FP | 4.18 | 18.6 | 4.18 | 0.15 |
| Water | | q.s. | 46.70 | |
| Total | 22.26 | 100 | 100.00 | 0.80 |
| Optional Ratio: Eudragit RS:RL **9:1** Coating layer: 5-20% (i.e. 10%)of core weight | | | | |
| | | | | |

| **Example Coating H** | parts (dry) | % (dry) | % (liquid) | mg/8mg core |
|---|---|---|---|---|
| Eudragit RL 12.5 | 1.28 | 17.75 | 30.93 | 0.14 |
| Eudragit RS 12.5 | 3.86 | 53.54 | 10.30 | 0.43 |
| Triethylcitrate | 0.52 | 7.21 | 0.52 | 0.06 |
| Syloid 244 FP | 1.55 | 21.50 | 1.55 | 0.17 |
| Acetone | q.s. | q.s. | 28.35 | |
| Isopropanol | q.s. | q.s. | 28.35 | |
| Total | 7.21 | 100.00 | 100.00 | 0.80 |
| Optional Ratio: Eudragit RS:RL **7.5:2.5** Coating layer: 5-20% (i.e. 10%) of core weight | | | | |
| | | | | |

| **Example Coating I** | Parts | % (dry) | parts (liquid) | mg/8 mg core |
|---|---|---|---|---|
| HPMC AS (Type MF) | 4.29 | 20 | 4.29 | 0.16 |
| Eudragit RS 30 D | 13.49 | 57.5 | 40.89 | 0.46 |
| Triethyl-citrate | 2.68 | 12.5 | 2.68 | 0.10 |
| Syloid 244 FP | 2.14 | 10 | 2.14 | 0.08 |
| Water | q.s. | q.s. | 50.00 | |
| Total | 22.60 | 100 | 100.00 | 0.80 |
| Optional Ratio: Eudragit RS: HPMC AS: **7.5 : 2.5** Coating layer: 5-20% (i.e. 10%) of core weight | | | | |

### 2.2.3 Diffusion coatings based on ethylcellulose polymer (with optional pore former)

The diffusion coating may contain ethylcellulose. The release rate of diffusion coats based on ethylcellulose may be controlled by varying one or more of the coating layer thickness (coating amount), the amount of hydrophilic coating compounds (e.g. plasticizers such as triethylcitrate, PEG 4000 or PEG 4000) and/or pigments/anti-sticking agents (e.g. colloidal silicium dioxide or Syloid 244 FP) and the amount of any pore-forming polymers.

Hydroxypropylmethylcellulose is an example of a suitable pore former and may be combined with ethylcellulose applied from organic coating solution or in combination with Aquacoat ECD dispersions (30% aqueous dispersion of ethylcellulose). The ratio of ethylcellulose and pore former may vary between 95: 5 and 30:70. Enteric polymers such as hydroxypropylmethylcellulose phthalate (HP 50) or hydroxypropylmethylcellulose acetate succinate (Aqoat) are also suitable pore formers, which suppress drug release in the stomach and control release in the intestinal juice with pH > 5.5. HP 50 can be combined with ethylcellulose coated from organic solution in the range of 5-50%. The coating layer is applied in the range of 5-30% of core weight, depending on the size and volume of the core pellet or minitablet.

| **Example Coating J** | parts | % (dry) | parts (liquid) | mg/8 mg core |
|---|---|---|---|---|
| Diffusion coat | | | | |
| Ethylcellulose | 6.75 | 75.00 | 6.75 | 0.60 |
| HPMC 3 cps | 0.75 | 8.33 | 0.75 | 0.07 |
| Aerosil 200 | 1.50 | 16.67 | 1.50 | 0.13 |
| Acetone | q.s. | q.s. | 45.50 | |
| Ethanol | q.s. | q.s. | 45.50 | |
| Total | 9.00 | 100.00 | 100.00 | 0.80 |
| Ethylcellulose : HPMC 3 cps = 9:1 Coating layer: 5-20% (i.e. 10%) of core weight | | | | |
| | | | | |

| **Example Coating K** | parts (dry) | % (dry) | parts (liquid) | mg/8 mg core |
|---|---|---|---|---|
| Diffusion coat | | | | |
| Ethylcellulose | 6.00 | 66.67 | 6.00 | 0.80 |
| HP 50 or Aqoat TYPE M | 1.50 | 16.67 | 1.50 | 0.20 |
| Aerosil 200 | 1.50 | 16.67 | 1.50 | 0.20 |
| Acetone | q.s. | q.s. | 45.50 | |
| Ethanol | q.s. | q.s. | 45.50 | |
| Total | 9.00 | 100.00 | 100.00 | 1.20 |
| Ethylcellulose : HP 50 OR AQOAT = 8:2 Coating layer: 5-20% (i.e. 15%) of core weight | | | | |
| | | | | |

| **Example Coating L** | parts (dry) | % (dry) | parts (liquid) | mg/8 mg core |
|---|---|---|---|---|
| Diffusion coat | | | | |
| HPMC 3 cps | 1.33 | 11.16 | 1.33 | 0.09 |
| Aquacoat ECD | 8.37 | 70.22 | 25.11 | 0.56 |
| Triethyl-citrate or | 2.22 | 18.62 | 2.22 | 0.15 |
| Water | q.s. | | 71.34 | |
| Total | 11.92 | 100.00 | 100.00 | 0.80 |
| Ethylcellulose : HPMC = 8.5: 1.5 Coating layer: 5-20% (i.e. 10%) of core weight | | | | |
| | | | | |

| **Example Coating M** | parts (dry) | % (dry) | parts (liquid) | mg/8 mg core |
|---|---|---|---|---|
| Diffusion coat | | | | |
| HPMC AS (MF) | 2.20 | 21.57 | 2.20 | 0.26 |
| Aquacoat ECD | 6.10 | 59.80 | 18.30 | 0.72 |
| Triethyl-citrate | 1.90 | 18.63 | 1.90 | 0.22 |
| Water. | q.s. | q.s. | 77.60 | |
| Total | 10.20 | 100.00 | 100.00 | 1.20 |
| Ethylcellulose : AQOAT = 7:3 Coating layer: 5-20% (i.e. 15%) of core weight | | | | |

### 2.3 Application of diffusion coatings to multiparticulate systems

The diffusion coat is applied to the minitablets or pellets using fluidized bed equipment with Wurster principle or Hüttlin-type equipment (turbojet) with a product temperature in the range of 28 to 45 °C. It is proposed to cure (temper) the coat applied from aqueous dispersion after coating for 1-5 hours at 40°C (Eudragit) -60°C (Aquacoat) in a tray dryer or fluidized bed equipment.

The final dosage form may be a stickpack or hard capsule filled with the multiparticulate formulation, or a disintegrating tablet which releases coated multiparticulate pellets

### 3. In vitro dissolution study

The dissolution profiles of the matrix tablets of Examples 10 to 20 are determined using USP I basket or paddle apparatus (Sotax AT 7 Smart). Dissolution tests are performed in triplicate using 1000 ml phosphate buffer (pH 6.8, SDS 0.2 %), at 37 °C and a rotational speed of either 50 rpm or 100 rpm. At predetermined intervals, samples are withdrawn from the dissolution medium, filtered through a membrane and analyzed spectrophotometrically. Equivalent amounts of fresh buffer are added to maintain a constant dissolution volume. In the case of Example 16, a paddle rotating at 50 rpm is used in the dissolution tester. In all other cases, a basket rotating at 100 rpm is used.

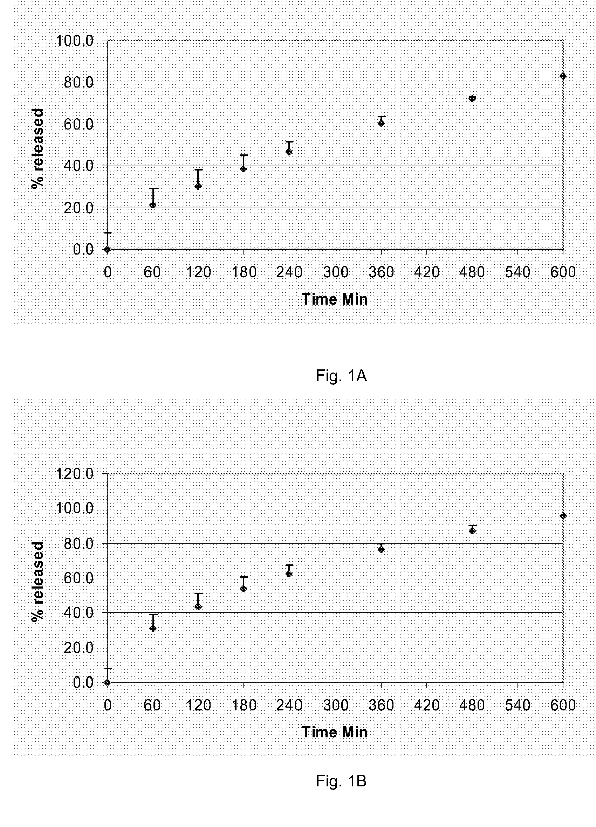

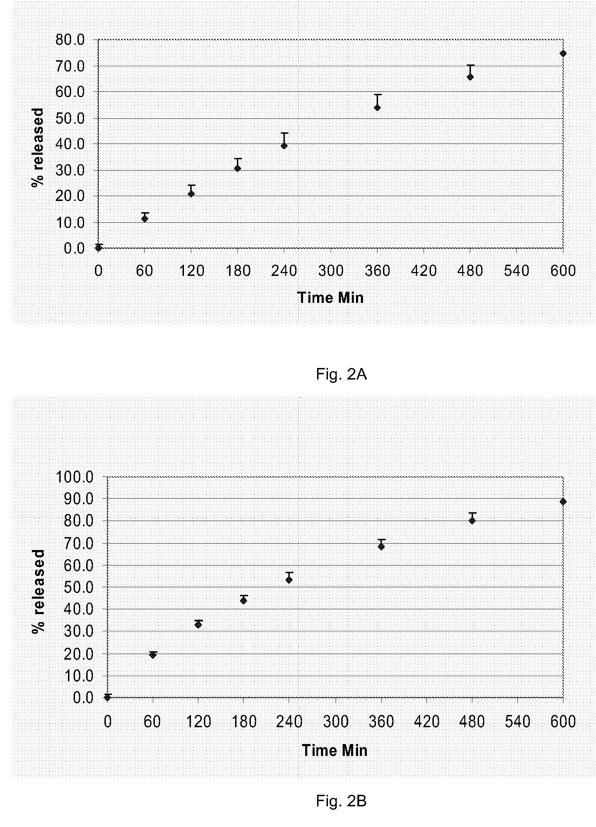

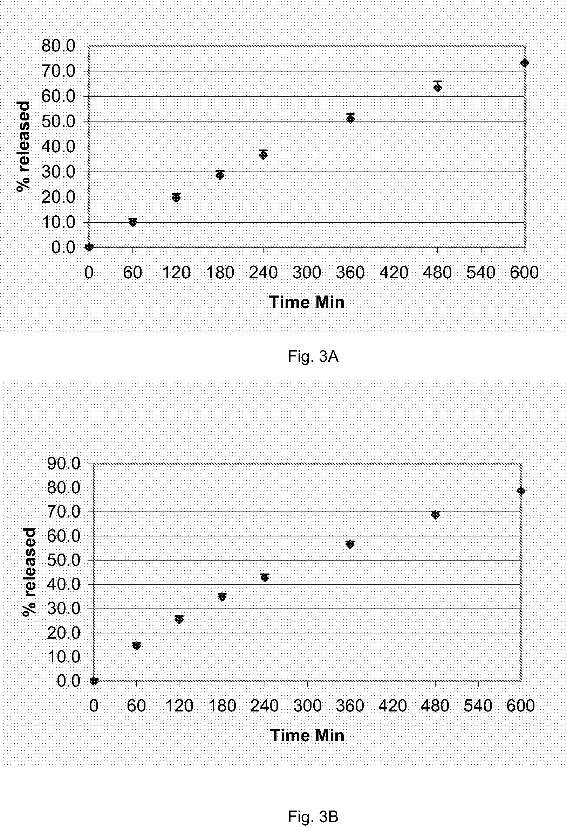

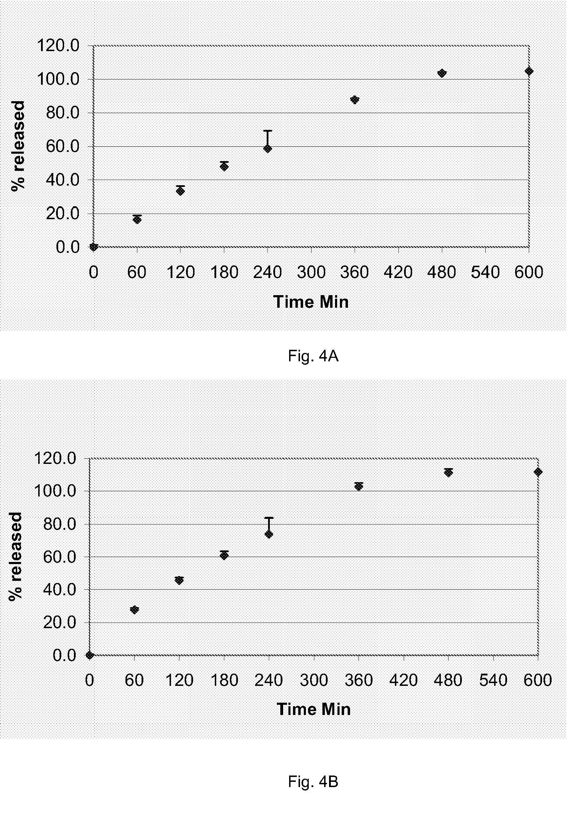

The dissolution data for the drug and acid (where present) of each composition are shown in the table below. The dissolution profiles of the matrix tablet formulations of Examples 10, 11, 13 and 18 are shown in Figs. 1, 2, 3 and 4 respectively. Part A of each Figure shows the drug dissolution profile, while Part B shows the acid dissolution profile. In each case, it can be seen that the drug and acid are released at substantially similar rates.

| **Example** | **Component** | **Dissolution Rate (% released at t =)** | | | | | | |
|---|---|---|---|---|---|---|---|---|
| | | **1h** | **2h** | **3h** | **4h** | **6h** | **8h** | **10h** |
| 10 | Drug | 21.1 | 30.4 | 38.7 | 46.8 | 60.6 | 72.0 | 82.9 |
| | Acid | 31.3 | 43.7 | 53.5 | 62.4 | 76.4 | 87.0 | 95.6 |
| 11 | Drug | 11.2 | 20.9 | 30.7 | 39.3 | 54.2 | 65.6 | 74.6 |
| | Acid | 19.1 | 32.8 | 44.1 | 53.5 | 68.4 | 80.2 | 88.5 |
| 12 | Drug | 21.4 | 32.4 | 41.6 | 48.8 | 61.5 | 70.7 | 77.9 |
| 13 | Drug | 9.9 | 19.5 | 28.5 | 36.6 | 51.0 | 63.4 | 73.3 |
| | Acid | 14.7 | 25.5 | 34.8 | 42.8 | 56.7 | 68.8 | 78.7 |
| 14 | Drug | 26.5 | 40.4 | 52.0 | 62.0 | 77.9 | 89.7 | 97.8 |
| 15 | Drug | 29.6 | 26.5 | 62.7 | 76.2 | 98.7 | 112.1 | 115.7 |
| 16 | Drug | 11.6 | 22.4 | 33.2 | 42.7 | 56.8 | 67.5 | 76.0 |
| | Acid | 26.3 | 42.7 | 57.1 | 68.8 | 87.1 | 99.5 | 106.9 |
| 17 | Drug | 13.0 | 22.0 | 31.3 | 39.4 | 53.5 | 65.2 | 76.5 |
| | Acid | 24.9 | 41.1 | 54.6 | 65.6 | 83.0 | 95.9 | 106.1 |
| 18 | Drug | 16.3 | 33.2 | 47.8 | 58.8 | 87.7 | 103.5 | 104.8 |
| | Acid | 27.6 | 45.6 | 60.8 | 73.9 | 102.8 | 111.2 | 111.7 |
| 19 | Drug | 15.0 | 25.0 | 34.0 | 42.0 | 54.0 | 65.0 | 74.0 |
| 20 | Drug | 21.0 | 35.0 | 47.0 | 56.0 | 78.0 | 98.0 | 105.0 |

## Claims

1. A solid pharmaceutical composition comprising:
(i) at least one acidic drug compound which is an angiotensin II receptor antagonist or a releasable form thereof;
(ii) at least one pH modifier; and
(iii) at least one pharmaceutically acceptable excipient.

2. The pharmaceutical composition according to claim 1, which comprises at least one matrix polymer, e.g. a water-insoluble polymer.

3. The pharmaceutical composition according to claim 2, wherein the matrix polymer contains at least one of the acidic drug compound and the pH modifier.

4. The pharmaceutical composition according to claim 3, wherein the matrix polymer contains the acidic drug compound and the pH modifier.

5. The pharmaceutical composition according to claim 3 or claim 4, wherein the matrix polymer contains a substantially uniform dispersion of the acidic drug compound and the pH modifier.

6. The pharmaceutical composition according to any of claims 3 to 5, wherein the matrix polymer comprises hydroxymethylpropylcellulose (HPMC).

7. The pharmaceutical composition according to any preceding claim, which comprises a core containing the acidic drug compound and pH modifier, and a coating thereon.

8. The pharmaceutical composition according to claim 7, wherein the coating comprises a diffusion coating.

9. The pharmaceutical composition according to claim 8, wherein the diffusion coating comprises a water-insoluble material.

10. The pharmaceutical composition according to claim 9, wherein the diffusion coating comprises one or more of ethylcellulose, acrylic acid ester and methacrylic acid ester.

11. The pharmaceutical composition according to any of claims 8 to 10, wherein the diffusion coating is porous.

12. The pharmaceutical composition according to any of claims 8 to 11, wherein the diffusion coating comprises a pore-forming agent.

13. The pharmaceutical composition according to claim 12, wherein the diffusion coating comprises a water-soluble polymer.

14. The pharmaceutical composition according to claim 13, wherein the diffusion coating comprises one or both of hydroxypropylmethylcellulose and polyethyleneglycol.

15. The pharmaceutical composition according to any preceding claim, which is adapted to provide a microenvironmental pH of 5 or less in at least a region of the gastrointestinal tract.

16. The pharmaceutical composition according to claim 15, which is adapted to provide a microenvironmental pH of 4 or less in at least a region of the gastrointestinal tract.

17. The pharmaceutical composition according to claim 15 or claim 16, which is adapted to provide a locally acidic environment in one or more of the duodenum, jejunum, ileum and colon.

18. The pharmaceutical composition according to claim 17, which is adapted to provide said microenvironmental pH in the jejunum, ileum and colon.

19. The pharmaceutical composition according to any preceding claim wherein the acidic drug compound is weakly acidic.

20. The pharmaceutical composition according to any preceding claim wherein the acidic drug compound has a pKa of 2.5 or more at 25 °C.

21. The pharmaceutical composition according to any preceding claim wherein the acidic drug compound has a pKa of 3 or more, for example 4 or more, at 25 °C.

22. The pharmaceutical composition according to any preceding claim wherein the acidic drug compound comprises one or more acidic groups.

23. The pharmaceutical composition according to claim 22 wherein the acidic drug compound comprises one or more carboxylic acid groups.

24. The pharmaceutical composition according to claim 22 or claim 23 wherein the acidic drug compound comprises one or more tetrazole groups.

25. The pharmaceutical composition according to any preceding claim, wherein the acidic drug compound is valsartan.

26. The pharmaceutical composition according to any preceding claim, which further comprises one or more other antihypertensive agents.

27. The pharmaceutical composition according to claim 26, which comprises one or more agents selected from diuretics, calcium channel blockers, beta-blockers and ACE inhibitors.

28. The pharmaceutical composition according to claim 27, which comprises hydrochlorothiazide.

29. The pharmaceutical composition according to any preceding claim wherein the pH modifier has a pKa of greater than 2.

30. The pharmaceutical composition according to any preceding claim wherein the pH modifier is an organic acid.

31. The pharmaceutical composition according to claim 30 wherein the pH modifier is selected from citric acid, fumaric acid, succinic acid, adipic acid and maleic acid.

32. The pharmaceutical composition according to any preceding claim wherein the pH modifier is fumaric acid.

33. The pharmaceutical composition according to claim 32 wherein the weight/weight ratio of pH modifier to acidic drug compound is between 0.025:1 and 2:1.

34. The pharmaceutical composition according to claim 33 wherein the weight/weight ratio of pH modifier to acidic drug compound is about 0.25:1.

35. The pharmaceutical composition according to any preceding claim wherein the acidic drug compound is valsartan and the pH modifier has a value for the most acidic pKa of less than 3.5

36. The pharmaceutical composition according to claim 35 wherein the pH modifier has a pKa of about 3.

37. The pharmaceutical composition according to claim 36 wherein the pH modifier comprises fumaric acid.

38. The pharmaceutical composition according to any preceding claim, which is in the form of a tablet, minitablet, granule or pellet.

39. Use of an acidic angiotensin II receptor antagonist or a releasable form thereof and a pH modifier for the preparation of a medicament for the treatment, prevention or delay of progression of hypertension, congestive heart failure or myocardial infarction.

40. Use according to claim 39 wherein the angiotensin II receptor antagonist is weakly acidic.

41. Use according to claim 39 or claim 40 wherein the angiotensin II receptor antagonist is valsartan.

42. Use according to any of claims 39 to 41 wherein the pH modifier is as defined in any of claims 29 to 37.

43. A method of orally administering an acidic drug compound which is an angiotensin II receptor antagonist or a releasable form thereof, said method comprising orally administering to a patient in need thereof a pharmaceutical composition according to any one of claims 1 to 38.

44. A method according to claim 43 wherein the acidic drug compound is valsartan.

45. A method according to claim 44 wherein the composition is administered for the treatment, prevention or delay of progression of hypertension, congestive heart failure or myocardial infarction.

46. An oral valsartan composition adapted to create a locally acidic environment in the intestine.

47. A composition according to claim 46, which is adapted to create a locally acidic environment in one or more of the jejunum, ileum and colon.

48. Use of a pharmaceutically acceptable acid for the manufacture of an oral medicament comprising an acidic drug compound which is an angiotensin II receptor antagonist or a releasable form thereof, wherein the pharmaceutically acceptable acid promotes protonation of the acidic drug compound in the intestine.

49. A method of enhancing the intestinal absorption of an acidic drug compound which is an angiotensin II receptor antagonist, comprising co-administering the acidic drug compound or a releasable form thereof with a pH modifier.
